(19) Europäisches Patentamt
European Patent Office
Office européen des brevets

(11) Publication number: **0 177 808 A1**

(12) **EUROPEAN PATENT APPLICATION**

(21) Application number: **85111704.4**

(22) Date of filing: **16.09.85**

(51) Int. Cl.⁴: **C 07 D 233/64**, A 61 K 31/415

(30) Priority: **21.09.84 JP 198124/84**

(43) Date of publication of application: **16.04.86 Bulletin 86/16**

(84) Designated Contracting States: **DE FR GB SE**

(71) Applicant: **Kanto Ishi Pharmaceutical Co., Ltd, 1-1, Nishi Shinjuku 2-chome, Shinjuku-ku Tokyo (JP)**

(72) Inventor: **Horiuchi, Jiro, No. 554-13, Kamifujisawa, Iruma-shi Saitama-ken (JP)**
Inventor: **Suzuki, Kazuo, No. 2-1-13, Taishidou Setagaya-ku, Tokyo (JP)**
Inventor: **Ito, Masayoshi, No. 1-27-22-303, Fujimidai, Kunitachi-Shi Tokyo (JP)**
Inventor: **Kato, Tetsuzo, No. 1-9-6, Kunimi, Sendai-shi Miyagi-ken (JP)**
Inventor: **Shitori, Yoshiyasu, No. 6-12-6-1307, Nishishinjyuku Shinjyuku-ku, Tokyo (JP)**

(74) Representative: **Klunker, Hans-Friedrich, Dr. et al, Patentanwälte Klunker . Schmitt-Nilson . Hirsch Winzererstrasse 106, D-8000 München 40 (DE)**

(54) **4-Methyl-5-thioformamidinomethylimidazole dihydrochloride.**

(57) The present invention provided a novel compound, 4-methyl-5-thioformamidinomethylimidazole dihydrochloride, shown by formula (I):

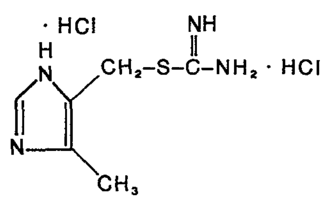

(I)

The compound (I) of the present invention is useful as an intermediate for an imidazole thioether derivative such as cimetidine which is an $H_2$-receptor antagonist.

EP 0 177 808 A1

KLUNKER · SCHMITT-NILSON · HIRSCH
PATENTANWÄLTE
EUROPEAN PATENT ATTORNEYS

KANTO ISHI PHARMACEUTICAL CO., LTD.
u.Z.: K 30 121S/6 16. September 1985

# 4-METHYL-5-THIOFORMAMIDINOMETHYLIMIDAZOLE DIHYDROCHLORIDE

## Background of the Invention

### Field of the Invention

The present invention relates to a novel intermediate useful for producing pharmaceuticals which have recently drawn attention as $H_2$-receptor antagonists, typically cimetidine.

### Description of the Related Art

Imidazole derivatives having a pharmaceutical activity or imidazole compounds which function as a histamine antagonist toward an $H_2$-receptor have recently drawn attention as treatment agents for peptic ulcer.

These imidazole compounds, however, are synthesized through various complicated processes or expensive intermediates. For example, Japanese Patent Public Disclosure No. 127361/1981 discloses a method for producing 4-[(2-aminoethyl)thiomethyl]-5-methylimidazole of the following formula:

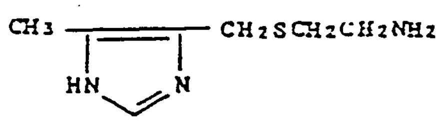

and its acid addition salt, which is an intermediate, this method being characterized by reacting a compound of the following formula:

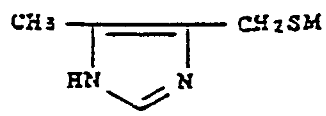

(wherein M indicates a hydrogen atom or an alkali metal) and 2-chloroethylamine or 2-bromoethylamine in a system comprising an aqueous solution of a strong base and an organic solvent in the presence of a phase transition catalyst of a quaternary ammonium salt or a quaternary phosphonium salt. This production method, however, uses relatively expensive raw materials, the reaction is complicated, and the yield of the intended compound is low. In addition, U.S. Patent No. 3,950,333 claims guanidine compounds useful as $H_2$-receptor antagonists and suggests several production methods, but does not describe the novel intermediate of the present invention nor the method for producing it.

Summary of the Invention

The present invention relates to a novel compound, 4-methyl-5-thioformamidinomethylimidazole dihydrochloride, shown by formula (I):

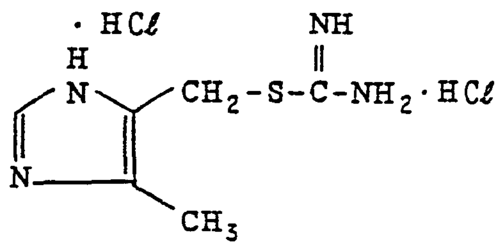

(I)

Description of the Preferred Embodiment

The compound of the present invention can be easily produced by reacting thiourea and 4-methyl-5-chloromethyl-imidazole in a solvent.

The molar ratio of thiourea and 4-methyl-5-chloromethyl-imidazole is 1:0.8-1.2. Methanol or ethanol is used as a solvent.

The reaction is carried out at a temperature in the range of 10-40$^{\circ}$C and preferably at room temperature.

The raw material of the present invention, thiourea, is available on the market at a low price. Another raw material, 4-methyl-5-chloromethylimidazole, can be quantitatively synthesized by reacting 4-methyl-5-hydroxymethylimidazole hydrochloride or 5-methyl-4-hydroxymethylimidazole with thionyl chloride.

The compound (I) of the present invention has the following advantages (usefulness);

That is, cimetidine (III) which is an $H_2$-receptor antagonist, can be easily synthesized by reacting the compound (I) with N-cyano-methylamino-2-chloroethylamino-azomethine (II) in the presence of an alkali.

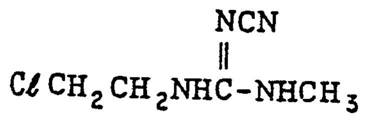

(II)

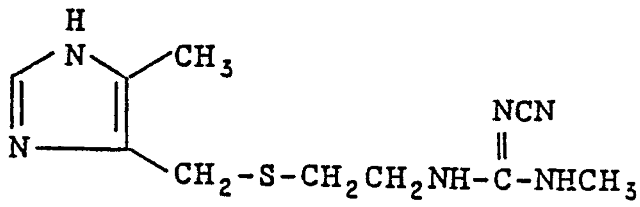

(III)

Furthermore, a novel imidazole thioether derivative shown by formula (V) can be synthesized by reacting the compound (I) with 4-chloro-acetoacetic ester (IV) in the presence of an alkali.

$ClCH_2COCH_2COOR$ (IV)

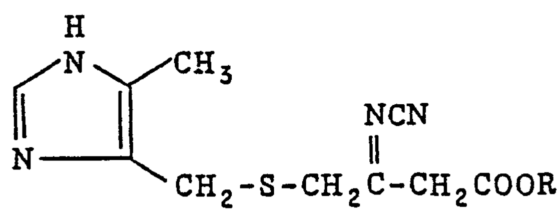

(V)

(wherein R indicates an alkyl group having 1 to 4 carbon atoms).

The above described imidazole thioether derivative is a pharmaceutical which has recently come into the spotlight as an $H_2$-receptor antagonist and is typically cimetidine.

However, conventional production is, for example, by a method which uses an expensive mercapto derivative (U.S. Patent No. 3,950,333) or a method which employs complicated

processes and a catalyst (Japanese Patent Public Disclosure No. 127361/1981). In addition, since the raw materials used in these methods are susceptible to oxidation, it is necessary to carry out the reactions in an inert gas flow. Thus the operation are complicated, and the yields are not always high.

In contrast, the compound (I) of the present invention is generally stable and is characterized in that reactivity does not appear until it is put in an aqueous solution in the presence of an alkali. Thus, the invention is characterized by the fact that it becomes unnecessary to use an inert gas when using the compound (I) of the present invention as the raw material and that a useful compound, the imidazole thioether derivative, can be synthesized under extremely simple operating conditions.

The compound (I) of the present invention is characterized by being capable of quantitative synthesis from thiourea and 4-methyl-5-chloromethylimidazole as well as by the fact that isolation and purification after the synthesis is unnecessary. This means that the product of the above described reaction can itself be used as the raw material for the synthesis of the imidazole thioether derivative.

Furthermore, the alkali which is present in the synthesis of the imidazole thioether derivative using the compound (I) of the present invention is sometimes preferably alkaline produced by sodium carbonate rather than by caustic alkali.

In addition, although this synthesis reaction generally progresses favorably only under agitation at room temperature, reaction carried out at a low temperature sometimes also progresses favorably.

The present invention will be further explained with reference to an example.

Example

15.24 g (0.2 mol) of thiourea was dissolved in 350 ml of ethanol and 33.4 g (0.2 mol) of 4-methyl-5-chloromethylimidazole hydrochloride (see Note) was added to the resulting solution, dissolved and stirred at room temperature for 3 hours. The solution was then concentrated to dryness under reduced pressure.

The dried substance was recrystallized from methanol to obtain 43.84 g of 4-methyl-5-thioformamidinomethylimidazole dihydrochloride in the form of colorless prisms. The m. p. was 200 - 202°C and the yield was 90%.

MS m/e: 171 $(M+1)^+$

IR $\nu_{max}^{KBr}$ $cm^{-1}$: 1630, 1440, 2500 ~ 3300

$^1H$-NMR(DMSO-$d^6$)$\delta$: 2.36 (3H, s, $-CH_3$), 4.84 (2H, s, $-CH_2-$), 9.10 (1H, s, imidazole ring) 9.63 (4H, m, -NH, -NH, $NH_2$)

$^{13}C$-NMR(DMSO-$d^6$)$\delta$: 8.8 (q, $-CH_3$) 24.0 (t, $-CH_2-$),

122.4 (s, imidazole $C_5$)

127.6 (s, imidazole $C_4$)

133.3 (d, imidazole $C_2$)

168.7 (s, $-S-\overset{\overset{NH}{\|}}{C}-NH_2-$)

Note: 4-Methyl-5-chloromethylimidazole used in the example was quantitatively obtained by reacting 4-methyl-5-hydroxymethylimidazole hydrochloride or 5-methyl-4-hydroxymethylimidazole with thionyl chloride.

We claim:

1. A compound shown by formula (I):

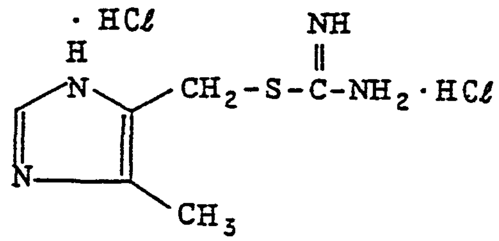

(I)

2. A method for producing a compound having formula (I):

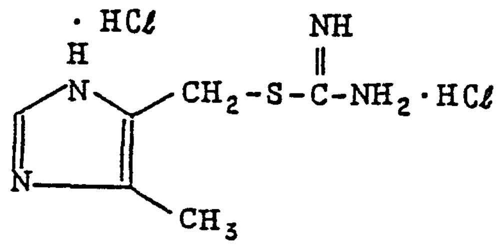

(I)

characterized by reacting thiourea and 4-methyl-5-chloro-methylimidazole in a solvent.

3. A production method according to claim 2, wherein the solvent is methanol.

4. A production method according to claim 2 or 3, wherein the reaction is carried out at room temperature.

0177808

European Patent Office

# EUROPEAN SEARCH REPORT

Application number

EP 85 11 1704

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| E | EP-A-0 129 033 (POLAROID)<br>* Page 14, formule IX; page 39, example IX *<br>----- | 1,2 | C 07 D 233/64<br>A 61 K 31/415 |
| | | | TECHNICAL FIELDS SEARCHED (Int. Cl.4) |
| | | | C 07 D 233/00<br>A 61 K 31/00 |

The present search report has been drawn up for all claims

| Place of search | Date of completion of the search | Examiner |
|---|---|---|
| THE HAGUE | 08-01-1986 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82